# EUROPÄISCHE PATENTSCHRIFT

(11) **EP 2 869 893 B1**
(45) Veröffentlichungstag und Bekanntmachung des Hinweises auf die Patenterteilung: **07.11.2018**
(21) Anmeldenummer: 13776553.3
(22) Anmeldetag: 02.07.2013
(51) Int. Cl.: A61N 2/00, A61N 1/40

(54) **VORRICHTUNG FÜR REPETITIVE NERVENSTIMULATION ZUM ABBAU VON FETTGEWEBE MITTELS INDUKTIVER MAGNETFELDER**
DEVICE FOR REPETITIVE NERVE STIMULATION IN ORDER TO BREAK DOWN FAT TISSUE BY MEANS OF INDUCTIVE MAGNETIC FIELDS
INSTALLATION DE STIMULATION NERVEUSE RÉPÉTITIVE POUR RÉDUIRE DES TISSUS ADIPEUX AU MOYEN DE CHAMPS MAGNÉTIQUES INDUCTIFS

(30) Priorität: 05.07.2012 DE 102012013534
(43) Veröffentlichungstag der Anmeldung: 13.05.2015
(73) Patentinhaber: MAGLETICS GmbH, 82049 Pullach im Isartal (DE)
(72) Erfinder: SOKOLOWSKI, Tobias, 82049 Pullach im Isartal (DE)
(74) Vertreter: Dehns
(86) Internationale Anmeldenummer: PCT/IB2013/001896
(87) Internationale Veröffentlichungsnummer: WO 2014/006504

(56) Entgegenhaltungen:
- WO-A1-00/13749
- WO-A1-2012/040243
- DE-A1- 10 062 050

## Beschreibung

Die vorliegende Erfindung nutzt den Effekt der Stimulation von Muskelkontraktionen durch berührungslose Induktion von elektrischen Feldern mittels pulsförmiger Magnetfelder im Gewebe. Pulsierende Magnetfelder sind dabei auch in der Lage durch Beeinflussung der elektrischen Ströme den Ionentransport anzuregen und den Stoffwechsel messbar zu erhöhen. Es kommt nachweislich zu einer Steigerung der Durchblutung und einer erhöhten Sauerstoffzufuhr. Diese Effekte werden derzeit unter dem Gesichtspunkt der Reaktivierung von Muskeln nach Krankheit oder Unfall medizinisch evaluiert und haben bereits zu entsprechenden Erfindungen geführt. So ist aus der DE 10 2007 044 445 A1 ein Trainingsgerät mit Magnetstimulation bekannt, bei dem in Verbindung mit Mitteln zur mechanischen Führung einer beabsichtigten Gelenkbewegung eines gelähmten Körperteils dieses zu Bewegungen angeregt wird. Aus der US 2005/0203332 A1 ist ein Gerät zur Behandlung von Osteoporose und anderen Muskel-Skelett-Erkrankungen bekannt, bei der der Patient auf einer Liege liegend am betreffenden Körperteil von einer zylindrischen, ein elektromagnetisches Feld erzeugenden Spule umgeben ist. Aus der US 6,213,933 B1 ist eine Vorrichtung und ein Verfahren zur Auflösung von Blutgerinseln in menschlichen Körperteilen entnehmbar, wobei der Patient auf einer länglichen Plattform liegt, über die eine quer verlaufende, längsbewegliche Halterung mit einer Wasser gekühlten Magnetfeldspule vom Butterfly Typ zur Stimulation angebracht ist. Zur Positionierung der Magnetfeldspule wird die Halterung einfach über das zu behandelnde Körperteil geschoben. Die Frequenz und Dauer der Stimulation wird mittels eines interaktiven Programms von einem PC gesteuert. Ein anderes, aus der US 2003/0158585 A1 bekanntes elektromagnetisches System nutzt zur Stimulation von Nerven, Muskeln und anderen Gewebeteilen des menschlichen Körpers ergonomische, der jeweiligen Körperteilkontur angepasste Stimulationsspulen in Form von flexiblen Flach- oder Zylinderspulen zur therapeutischen Behandlung.

Darüber hinausgehend - und bisher nicht genutzt -führt eine Muskelreizung mittels Magnetfeldstimulation zu einem Abbau von Fettgewebe in der Muskelumgebung, wie der Anmelder durch zahlreiche Versuche, insbesondere an adipösen und muskulösen Probanden, belegen konnte. Bei schlanken Probanden ergibt sich dabei ein Muskelaufbau ohne signifikante Gewichtsabnahme.

Zwar existieren bereits Vorrichtungen und Verfahren zur Behandlung von Fettleibigkeit bzw. Übergewicht mittels pulsierender Magnetfelder, diese benötigen aber neben der Feld erzeugenden Spule entweder noch einen mit der Körperoberfläche in Kontakt stehenden Permanentmagneten (DE 100 62 050 A1) oder wirken indirekt über ein die Schilddrüse aktivierendes Magnetfeld (DE 10 2009 043 728 A1) und müssen mittels eines Halsbandes ebenfalls am Körper angebracht werden.

Das Dokument WO-A-00/13749 offenbart den nächstliegenden Stand der Technik.

Unter Ausnutzung der zuvor genannten Erfahrung und Erkenntnisse verbleibt somit als Aufgabe der Erfindung, eine Vorrichtung und ein Verfahren für repetitive Nervenstimulation zum Abbau von Fettgewebe mittels induktiver Magnetfelder zu schaffen, die eine leichte patientenbezogene Einstellbarkeit und Steuerung erlauben, um Fettgewebe in definierten Körperregionen, wie Bauch, Gesäß oder Oberschenkel, gezielt und ohne Körperkontakt zu reduzieren.

Diese Aufgabe wird durch die im Patentanspruch 1 beanspruchte Vorrichtung
gelöst. Vorteilhafte Ausgestaltungen der Erfindung sind Gegenstand von Unteransprüchen.

Vorteile der Erfindung bestehen insbesondere in der berührungslosen Induktion der Reizung, der damit verbundenen geringen Schmerzreizung, die bei alternativer elektrischer Reizung auftritt, der Großflächigkeit der Reizung und der auf den individuellen Körperbau des Patienten abstimmbaren Positionierung der reizerzeugenden Spule.

Wesentliche Bestandteile des Gerätes sind eine großflächige stromdurchflossen Magnetfeldspule, im Weiteren Stimulationsspule genannt, die auf einem Ständer befestigt ist. Für die Behandlung von Bauch, Gesäß und Oberschenkeln sind verschiedene Spulenformen vorhanden

Die Stimulationsspule erzeugt in ca. 5 cm vor der Spulenoberfläche Magnetfelder mit Spitzen in der magnetischen Flussdichte von 0.01 T bis 0,1 T. Das Magnetfeld ist dabei zeitlich veränderlich und besteht aus biphasigen oder monophasigen Pulsen mit einer Pulsdauer T von 100 µs bis 300 µs. Die Wiederholfrequenz der Pulse (Stimulationsfrequenz fₚ) beträgt 10 Hz bis 30 Hz. Mittels Induktion werden am Reizort im Gewebe maximale elektrische Feldstärken von 0.1 V/cm bis 1 V/cm erreicht. Das Magnetfeld der Spule ist betragsmäßig über das zu behandelnde Gewebevolumen näherungsweise örtlich konstant. Parameter einer typischen Spule sind in Tabelle 1 zusammengestellt.

**Tabelle 1: Übersicht zu den Parametern der Spule für den Bauchbereich**

| **Größe** | **Wert** |
|---|---|
| Länge des Leiters | 200 cm |
| Querschnittsfläche | 1 cm² |
| Abstand benachbarter Spulenleiter | 1 cm |
| Maximale Stromamplitude I | 1000 A |
| Max. magn. Flussdichte B, 5 cm vor der Spulenoberfläche | 0.01 T |
| Induzierte elektrische Feldstärke (maximal), E | 0.1 V cm⁻¹ |
| Kräfte zwischen benachbarten Spulenleitern | 0,2 Ncm⁻¹ |
| Induktivität der Spule, L | 15 10⁻⁶ H |
| Pulsfrequenz, f | 5 10³ Hz |
| Induktiver Widerstand Z_{L} bei f | 470 m Ohm |
| Induktiver Spannungsabfall bei I | 470 V |
| Ohmscher Widerstand, R | 36 m Ohm |
| Stimulationsfrequenz, f_{Stim} | 30 Hz |
| Joulsche Verlustleistung, P | 120 W |

Die Ansteuerung der Stimulationsspule erfolgt über einen Pulsgenerator (Stimulator), der getrennt vom Ständer aufgestellt ist. Es kann ein derzeit als Prototyp in der medizinischen Forschung verwendeter Stimulator (IMETUM, Zentralinstitut für Medizintechnik, Technische Universität München, Abschlussbericht: "Funktionelle, periphere Magnetstimulation der Motorik in Patienten mit zentralen Paresen, insbesondere halbseitigen Lähmungen", 2011) mit einer Pulslänge von 160 µs (biphasig) eingesetzt werden. Die Kapazität eines Kondensators wird dabei an die Induktivität der Stimulationsspule angepasst, um die Resonanzfrequenz eines LC-Schwingkreises, bestehend aus der Stimulationsspule und dem Kondensator, auf die der Pulsdauer entsprechenden Frequenz abzustimmen. Die erforderlichen elektrischen Felder bedingen hohe Ströme durch die Spule im Bereich von 500 A bis 6000 A.

Die Großflächigkeit der Spule gestattet einen offenen und nicht vergossenen Aufbau. Dadurch kann die erhebliche anfallende Joulsche Wärme in der Spule mittels eines Luftgebläses abgeführt werden. Luftführung ist zur effektiven Kühlung vorgesehen. Mittels Filtermatten wird eine Kontamination und Staubverwirbelung vermieden.

Die Behandlungszeiten betragen 1 min bis 45 min. Die langen Zeiten können ohne Überhitzung der Spule aufgrund der effektiven Kühlung und der Spulenkonstruktion erreicht werden.

Die Spule selbst kann aufgrund ihrer Größe als selbsttragend aus massivem Metall (z.B. Kupfer oder Aluminium) gefertigt werden. Dadurch wird die Wärmeableitung aus dem Spulenleiter an seine Oberfläche unterstützt. Der Querschnitt des Leiters beträgt 1 cm² bis ca. 2 cm². Dieser im Vergleich zu dem von Spulen, die in der ortselektiven Magnetfeldstimulation (fokussierend) eingesetzt werden, große Querschnitt des Spulenleiters reduziert den ohmschen Widerstand, wodurch die Joulsche Wärme reduziert wird.

Alternativ kann die Spule zur Vermeidung von Skin- und Proximity-Effekten auch in konventioneller Technik aus Hochfrequenzdrähten erstellt werden. Eine Abschätzung der Skintiefe zeigt aber, dass dies bei einer Pulsdauer von ca. 200 µs nicht erforderlich ist.

Bei massiver Bauweise ist ggf. auch die Verwendung von Hohlleitern, die mittels einer Kühlflüssigkeit gekühlt werden denkbar.

Die Stimulationsspule ist in einem Gehäuse aus Kunststoff gekapselt. Das Gehäuse gewährleistet die Berührspannungssicherheit und gleichzeitig die Führung der Kühlluft.

Das Luftgebläse erzeugt einen Kühlluftstrom, der über im Kunststoffgehäuse der Stimulationsspule befindliche, mit Filter versehene Lufteintrittsöffnungen und Luftführungselemente an die Spule gelangt und über eine Luftführung aus Kunststoff, die um die elektrischen Verbindungsleitungen zur Spule angeordnet ist, den Luftaustrittsöffnungen des Kühlgebläses zugeführt wird. Das Luftgebläse kann dadurch rückseitig am Ständer und außerhalb des Magnetfeldes der Stimulationsspule angeordnet werden, wodurch eine Beeinträchtigung des elektrischen Gebläsemotors vermieden wird.

Aufgrund der hohen Ströme treten hohe mechanische Kräfte zwischen den Leiterabschnitten der Stimulationsspule und den Verbindungsleitungen auf. Diese werden durch abstandshaltende, keramische Halteelemente abgefangen.

Die elektrischen Verbindungsleitungen zur Stimulationsspule im Ständer sind massiv und ohne bewegliche Kabel ausgeführt. Dies erlaubt eine optimale Wärmeabgabe an die Luft, Abfangen der magnetischen Kräfte und eine hohe Betriebssicherheit.

Die Stimulationsspule ist auf einer Halterung montiert, die durch die massiven Stromzuführungen selbst gebildet wird. Diese positioniert die Spule in ausreichendem Abstand vom Ständer; die durch die Spule im Ständer induzierten, unerwünschten Wirbelströme werden so minimiert.

Eine Stimulationsspule kann nach Öffnung einer tordial und axial gelagerten Verschlusshülse mit Bajonettverschluss und Lösen zweier Schraubverbindungen für die Verbindungsleitungen leicht ausgewechselt werden. Dies ist relevant, da für unterschiedliche Körperregionen (Bauch, Oberschenkel, Gesäß) verschiedene, optimierte Spulenformen mit unterschiedlichen Gehäusen eingesetzt werden.

Die Positionierung der Stimulationsspule relativ zum Patienten kann mittels eines höhenvariablen Podestes variiert werden. Dadurch können die Verbindungsleitungen zur Stimulationsspule starr ausgeführt werden und Änderungen der Induktivität und Zuleitungswiderstände bei Veränderung der Positionierung vermieden werden.

Alternativ könnte eine Positionierung der Stimulationsspule am Ständer durch Klemmverschlüsse in vertikalen Schienen ausgeführt werden.

Die Stimulation der Muskelkontraktion kann mittels einer Rückkopplung optimiert werden. Dazu werden mittels einer im Ständer integrierten Kamera die durch eine Stimulationsspule ausgelösten Kontraktionen beobachtet und entsprechende Signale an einen Computer (z.B. Laptop) übertragen. Dieser steuert mittels eines entsprechenden Programms in Abhängigkeit der empfangenen Signale die am Ausgang des Pulsgenerators liegenden elektrischen Impulse bezüglich Pulsform, Pulsdauer, Pulsamplitude und Behandlungszeit.

Ein Ausführungsbeispiel der Erfindung, das weitere Vorteile und Besonderheiten erkennen lässt, wird in den Figuren 1 bis 8 dargestellt und im Folgenden näher beschrieben.

Es zeigen:
- Figur 1:: Gesamtansicht der Vorrichtung für repetitive Nervenstimulation zum Abbau von Fettgewebe mittels induktiver Magnetfelder
- Figur 2:: Detailansicht der Ständer/Stimulationsspulen-Verbindungsanordnung
(a) perspektivische Ansicht
(b) Schnittdarstellung
- Figur 3:: Leitungskopplung für Hin- und Rückleiter innerhalb der Ständer/Stimulationsspulen-Verbindungsanordnung
(a) Schnittdarstellung
(b) perspektivische Darstellung im gekoppelten Zustand
- Figur 4:: Stimulationsspule zur Bauchbehandlung
(a) perspektivische Gehäuseansicht von vorn (Behandlungsseite) mit geöffnetem Schutzdeckel
(b) perspektivische Gehäuseansicht von hinten (ständerseitig) mit sichtbaren Leitungskopplungen
- Figur 5:: Stimulationsspule zur Behandlung der Gesäßregion
(a) Gehäuseansicht von vorne (Behandlungsseite)
(b) Gehäuseansicht von der Seite
(c) Gehäuseansicht in Aufsicht
(d) Spulenverlauf im Inneren des Gehäuses vom Behandelnden aus gesehen
- Figur 6:: Stimulationsspule zur Oberschenkelbehandlung
(a) Gehäuseansicht von vorne (Behandlungsseite)
(b) Gehäuseansicht von der Seite
(c) Gehäuseansicht in Aufsicht
(d) Spulenverlauf im Inneren des Gehäuses vom Behandelnden aus gesehen
- Figur 7:: Prinzipschaltbild eines bipolaren Pulsgenerators
- Figur 8:: idealisierter Stromverlauf in einer Stimulationsspule bei bipolarer Ansteuerung durch den Pulsgenerator

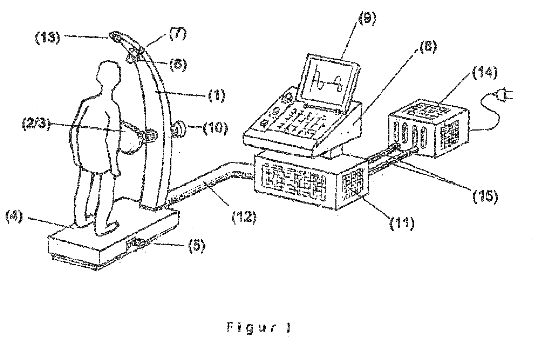

Figur 1 zeigt in Gesamtansicht die beanspruchte Vorrichtung für repetitive Nervenstimulation zum Abbau von Fettgewebe mittels induktiver Magnetfelder mit einer an einem Ständer (1) auswechselbar befestigten, in einem Kunststoffgehäuse (2) aufgenommenen Stimulationsspule (3), einem höhenvariablen Podest (4) mit Fußpumpe (5) zur individuell auf den Körper eines Patienten einstellbaren Positionierung gegenüber der Stimulationsspule (3), einer im Ständer (1) integrierten schwenkbaren Kamera (6) mit Einstellring (7) zur Beobachtung ausgelöster Muskelkontraktionen und zur Rückkopplung an einen in einer Bedienungskonsole (8) integrierten Laptop(9) mit Steuerungssoftware zur Computer-unterstützten Stimulationsoptimierung, einem Luftgebläse (10) zur Kühlung der Magnetfeldspule (3), einem Pulsgenerator (11) zur elektrischen Ansteuerung der Magnetfeldspule (3), einem abgeschirmten Kabelkanal (12) für die notwendigen elektrischen Verbindungsleitungen zwischen dem Ständer(1) und dem Pulsgenerator (11) bzw. der Bedienungskonsole (8) und einen Strahler (13) zur korrekten Ausleuchtung der von der Kamera (6) erfassten Körperregion. Es wird darauf hingewiesen, dass der Pulsgenerator (11) in vorteilhafter Weise direkt am Ständer (1) angeordnet werden kann, um Leitungsverluste zu minimieren. In diesen Fall entfällt mithin der dargestellte Kabelkanal (12). Ebenfalls dargestellt ist das zur Spannungsversorgung der Vorrichtung gehörende, aus Gründen der hohen Wärmeentwicklung separat gestaltete und aufgestellte Netzgerät (14) mit Ausgangsspannungen zwischen 500 und 1000 V und den zugehörigen Verbindungskabeln (15).

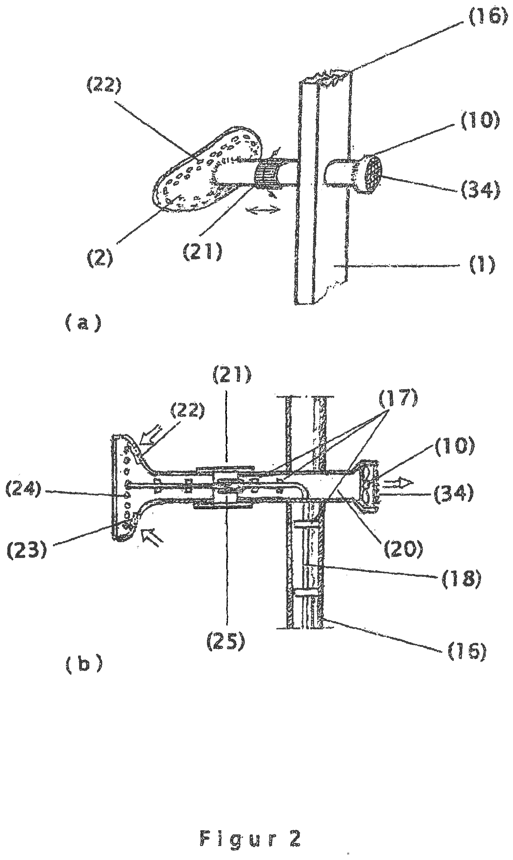

Der im oberen Bereich dem magnetischen Feldlinienverlauf annähernd angepasste Ständer (1) besteht im Wesentlichen aus einem aus Kunststoff gefertigten Hohlrahmen (16) mit im Inneren befindlichen keramischen Halteelementen (17) zur genauen Positionierung der beabstandeten Verbindungsleitungen (18, 19) mit einem Leitungsquerschnitt von 1,5 cm² (Fig. 2a, b). Diese Verbindungsleitungen (18, 19) übernehmen die Stromführungen zwischen Pulsgenerator (11) und Stimulationsspule (3). Sie können auch als Hohlleiter ausgebildet sein. Etwa in Höhe der Taille eines Patienten weist der Ständer (1) einen fest eingebauten, horizontal und quer durch den Ständer (1) verlaufenden rohrförmigen Luftführungskanal (20) mit den Verbindungsleitungen (18, 19) im Inneren auf. Die Verbindungleitungen (18, 19) sind vom Luftführungskanal (20) durch abgedichtete Bohrungen in den Ständer (1) geführt.

Figur 1 zeigt weiterhin die ebenfalls im Ständer (1) vorhandene Kamera (6), die mittels eines Einstellringes (7) so positioniert werden kann, dass die behandelte Körperregion zuverlässig erfasst wird. Zum Ausleuchten der von der Kamera (6) erfassten Körperregion dient ein Strahler (13) am oberen Ende des Ständers (1).

Das höhenvariable Podest (4) kann eine mechanische Hubvorrichtung aufweisen, die mittels Fußhebel (5) betrieben und arretiert wird. Denkbar ist eine vertikale Stütze. Auch können vertikal übereinander angeordnete Scheren mit einer horizontal dazwischenliegenden Gewindespindel zur Höhenveränderung geöffnet oder geschlossen werden, wobei die Gewindespindel von Hand oder von einem Elektromotor gedreht wird. Als weitere konstruktive Höhenverstellung des Podestes (4) kann ein hydraulischer Hebezylinder mit Fußpumpe gewählt werden.

Figur 2a zeigt die Ständer/Stimulationsspulen-Verbindungsanordnung im Detail mit dem Ständer (1), dem Luftführungskanal (20) und dem Luftgebläse (10) zur Kühlung der Verbindungsleitungen (18, 19) sowie der Stimulationsspule (3). Eine axial verschieb- und verdrehbar gelagerte Verschlusshülse (21) mit Bajonettverschluss deckt den Anfang des Luftführungskanals (20) und das Ende der Stimulationsspule (3) als Berührungsschutz der Leitungskopplungen ab.

Figur 2b zeigt in Schnittdarstellung die Ständer/Stimulationsspulen-Verbindungsanordnung. Der Luftführungskanal (20) im Hohlrahmen (16) des Ständers (1) weist an dem dem Patienten abgewandten Ende das Luftgebläse (10) mit Luftauslassöffnungen (34) auf, welches Luft über die Lufteinlassöffnungen (22) im Kunststoffgehäuse (2) der Stimulationsspule (3), Filtermatten (23), vorbei an nicht dargestellten Luftführungselementen, den Windungen (24) der Stimulationsspule (3) und den Verbindungsleitungen (18, 19) zur Kühlung absaugt. Auch ist die Leitungskopplung (25) für Hin- und Rückleiter der Verbindungsleitungen (18, 19) dargestellt.

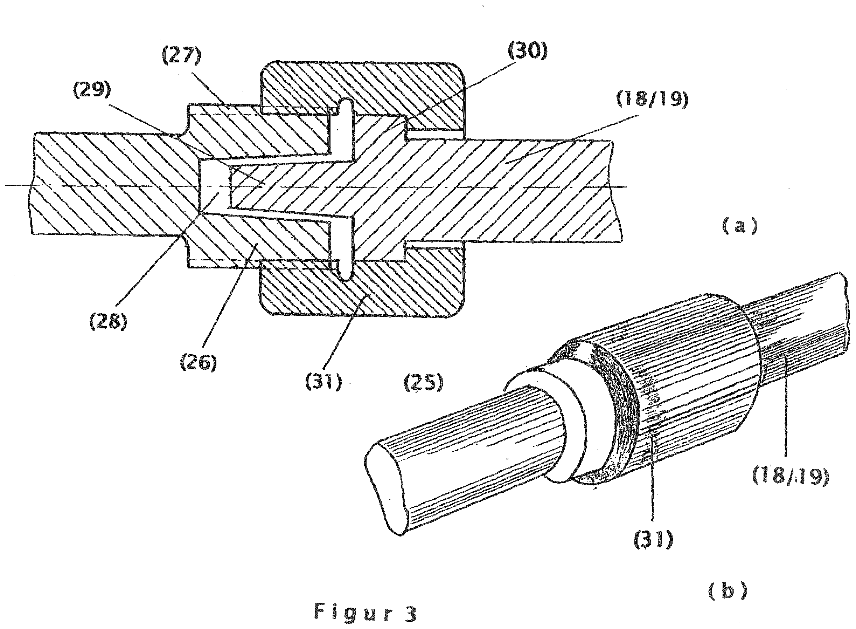

Figur 3a zeigt die Leitungskopplung (25) für Hin- und Rückleiter im Schnitt. Jedes Ende eines Leiters in der Stimulationsspule (3) weist einen verdickten, zylindrischen Endabschnitt (26) mit Außengewinde (27) auf. In diesen Endabschnitt (26) ist ein horizontaler, trapezförmiger Schlitz (28) gefräst. In diesen greift eine trapezförmige Lasche (29) ein, welche von einem verdickten, zylinderförmigen Endabschnitt (30) einer der Verbindungsleitungen (18) oder (19) ausgeht. Eine Schraubmuffe (31) mit Innengewinde, welche jeweils einen der verdickten Endabschnitte (30) der Verbindungsleitungen (18) oder (19) umgreift, ist auf das Außengewinde (27) jeweils eines Endabschnittes (26) aufgeschraubt und verbindet die Verbindungsleitungen (18, 19) mit der Stimulationsspule (3). Durch diese zwei steckbaren und fest verschraubbaren Leitungskopplungen wird die Stimulationsspule (3) von den Verbindungsleitungen (18, 19) getragen. Diese spezielle Schraubverbindung hat gleichzeitig die Funktion des elektrischen Leitens. Da die konischen Flanken des trapezförmigen Schlitzes (28) an den Flanken der trapezförmigen Lasche (29) großflächig anliegen, ergibt sich zudem ein geringer Übergangswiderstand.

Figur 3b zeigt die für Hin- und Rückleiter verwendete Leitungskopplung im verschraubten Zustand in perspektivischer Darstellung.

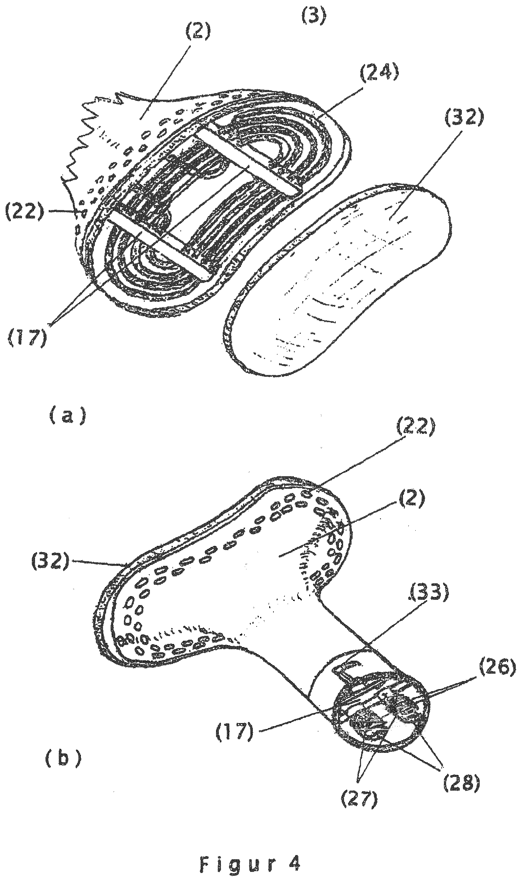

Figur 4a zeigt beispielsweise die Stimulationsspule (3) zur Bauchbehandlung in perspektivischer Darstellung von vorn (Behandlungsseite). Zu erkennen ist ein abgenommener aufklemmbarer Schutzdeckel (32) und ein annähernd trompetenförmiges Kunststoffgehäuse (2). An der Innenseite dieses trompetenförmigen Kunststoffgehäuses (2) sind mehrere keramische Halteelemente (17) zur Aufnahme der Windungen (24) der Stimulationsspule (3) vorhanden. Die Enden der Windungen (24) der Stimulationsspule (3) sind parallel geführt zu den verdickten Endabschnitten (26). Die Windungen (24) für die Bauchbehandlung selbst verlaufen annähernd oval und decken einen Bereich von ca. 20 cm x 30 cm ab. Auch sind die Lufteinlassöffnungen (22) erkennbar.

Figur 4b zeigt die Stimulationsspule (3) zur Bauchbehandlung in perspektivischer Darstellung von hinten (ständerseitig). Deutlich erkennbar sind die verdickten Endabschnitte (26) mit den Außengewinden (27), den jeweiligen trapezförmigen Schlitzen (28), keramische Halteelemente (17) und eine Nut (33) im Kunststoffgehäuse (2) der Stimulationsspule (3) zum Führen der axialen und verdrehenden Bewegung der Verschlusshülse (21).

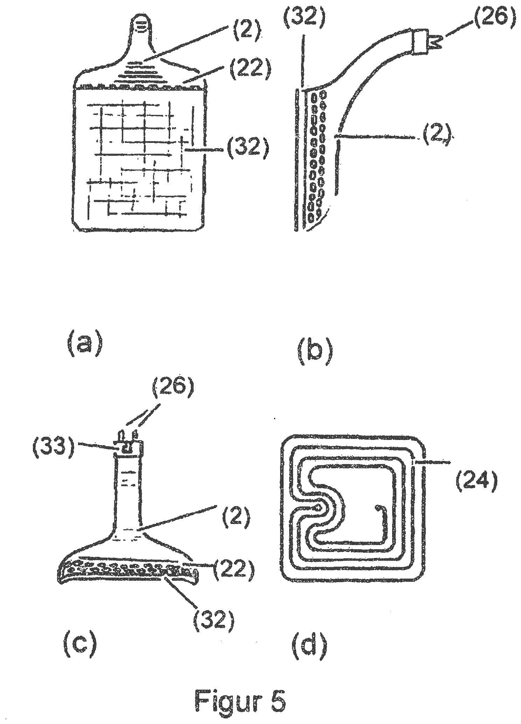

Figuren 5a, 5b, 5c, 5d zeigen verschiedene Darstellungen einer Stimulationsspule (3) zur Behandlung der Gesäßregion; (a) die Gehäuseansicht von vorn, (b) die Gehäuseansicht von der Seite, (c) die Gehäuseansicht in Aufsicht und (d) den Spulenverlauf im Inneren des Gehäuses vom Behandelten aus gesehen. Diese Stimulationsspule (3) hat den gleichen konstruktiven inneren Aufbau wie die Stimulationsspule (3) zur Bauchbehandlung und unterscheidet sich im Wesentlichen nur durch die äußere Form des Gehäuses. Die Behandlungsseite des Gehäuses hat eine abgerundete quadratisch Form von ca. 15 cm x 15 cm. Der Schutzdeckel (32) ist leicht konkav gewölbt. Die Simulationsspule (3) selbst besitzt ebenfalls quadratisch verlaufende, angepasste Windungen (24). Die Rückseite des Kunststoffgehäuses (2) ist schwanenhalsförmig geformt, damit die Stimulationsspule (3) ein Stück unterhalb der Taille des Patienten wirkt.

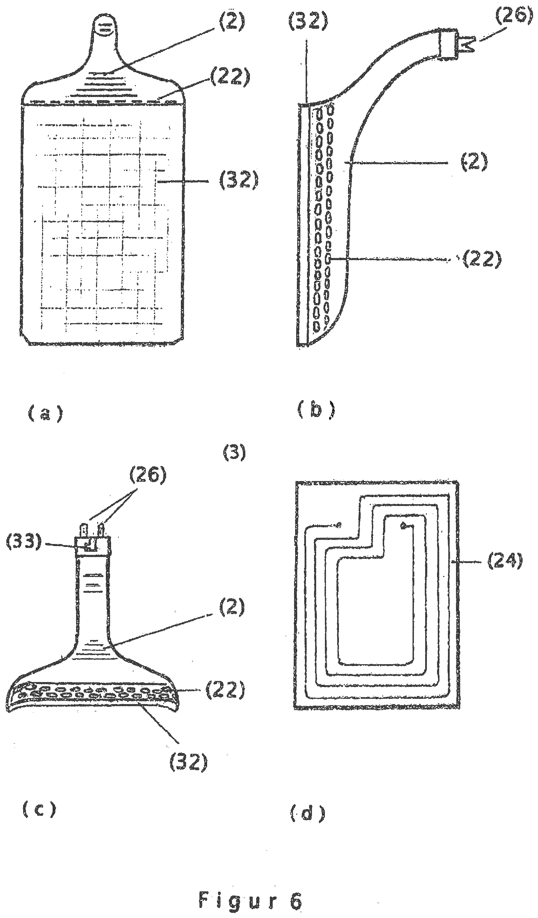

Figuren 6a, 6b, 6c, 6d zeigen verschiedene Darstellungen einer Stimulationsspule (3) zur Oberschenkelbehandlung; (a) die Gehäuseansicht von vorn, (b) die Gehäuseansicht von der Seite, (c) die Gehäuseansicht in Aufsicht und (d) Spulenverlauf im Inneren des Gehäuses vom Behandelten aus gesehen. Grundsätzlich ist diese Stimulationsspule (3) aufgebaut wie die für die Gesäßregion mit dem Unterschied, dass die Behandlungsseite eine abgerundete rechteckige Form von ca 10 cm x 20 cm abdeckt, wobei der Schutzdeckel eine in vertikaler Richtung verlaufende, leicht konkave Wölbung aufweist.

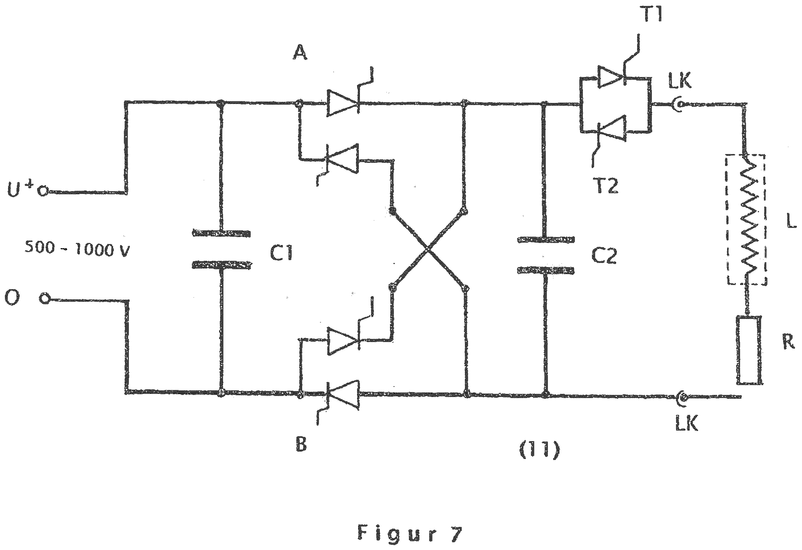

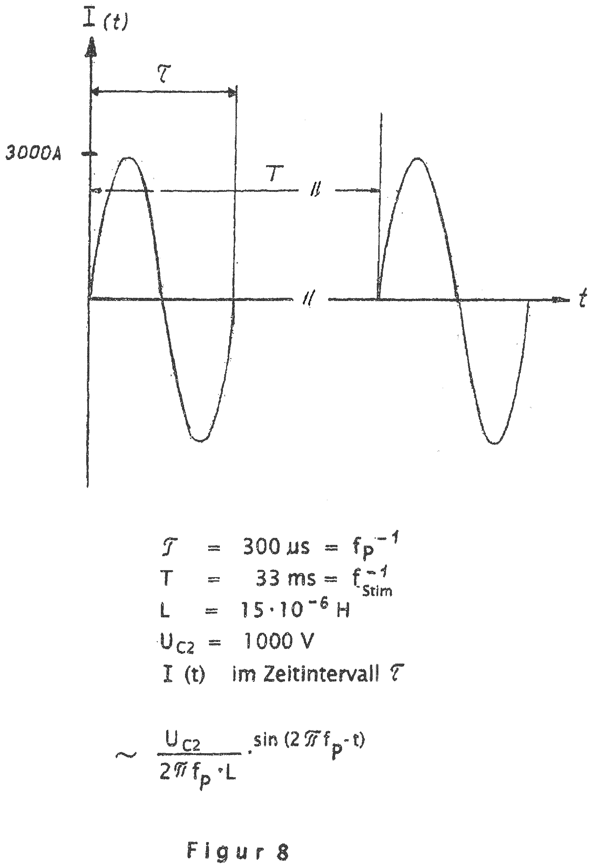

Figur 7 zeigt beispielhaft ein Prinzipschaltbild eines verwendeten herkömmlichen Pulsgenerators (11), der erst in Verbindung mit der Stimulationsspule (L) (siehe Leitungskopplungen LK) eine eigentliche Stimulationspulserzeugungseinheit ergibt. Vom separaten Netzteil (14) kommende Spannungen zwischen 500 V und 1000 V werden in Stufen von 50 V mittels elektronischer Schalter (hier nicht dargestellt), die entweder per Hand von der Bedienungskonsole (8) aus oder vom Computer (9) gesteuert werden, selektiert und an den am Eingang des Pulsgenerators (11) liegenden Ladekondensator (C1) zur Speicherung angelegt. Die gewählte Aufladspannung dieses Ladekondensators (C1) bestimmt im Wesentlichen die Stimulationsleistung und damit die Amplitudenhöhe des zu erzeugenden Stimulationspulses. Über die Thyristorschaltungen A und B wird der eigentliche Energiespeicher, der Pulskondensator (C2), auf- bzw. nachgeladen. Der Pulskondensator (C2) bildet mit der Stimulationsspule (L) im Prinzip einen Schwingkreis, der noch durch die ohmschen Widerstände der Verbindungsleitungen und -kopplungen, hier als ohmscher Widerstand (R) pauschal eingezeichnet, gedämpft wird. Repetitive Entladungen des Pulskondensators (C2) und damit die Stimulationsfrequenz **f_{Stim}** werden mittels zweier antiparallel geschalteter, starkstromtauglicher Thyristoren (T1, T2) gesteuert, die den Pulskondensator (C2) mit der Stimulationsspule (L) verbinden. Aufgrund der antiparallelen Schaltung der Thyristoren (T1, T2) können sinusförmige biphasige Spannungs- und Strompulse generiert werden (Figur 8 und zugehörige Beschreibung). Deren Impulsdauer 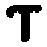 ist jeweils durch das Hinzu- oder Abschalten von zum Pulskondensator (C2) parallel geschalteten Kondensatoren (hier nicht dargestellt) einstellbar, da sich hierdurch die im Schwingkreis befindliche Gesamtkapazität und mithin die Schwingkreisfrequenz **f_{P}** (**f_{P}** = 1/(2π √LC) ändern lässt. Sowohl die Thyristoransteuerung zur Auswahl der Stimulationsfrequenz **f_{Stim}** als auch die Anzahl parallel geschalteter Kondensatoren zur Bestimmung der Pulsdauer 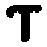 sind an der Bedienungskonsole (11) per Hand voreinstellbar oder werden vom Computer (9) kontrolliert.

In Figur 8 ist beispielhaft ein idealisierter Stromverlauf in einer Stimulationsspule (3) bei biphasiger Ansteuerung durch den Pulsgenerator (11) dargestellt. Hierbei wurde eine Stimulationsspuleninduktivität von 15×10⁻⁶ H, eine Aufladspannung U_{C2} von 1000 V und ein biphasiger Stimulationspuls mit einer Pulsdauer 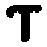 von 300 µs angenommen. Nach dem Durchschalten des Thyristors (T1) (Figur 7) ergibt sich eine positive Halbwelle eines sinusförmigen Stromverlaufs mit einer maximalen Amplitude von circa 3000 A. Zum Zeitpunkt 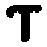 /2, also nach Ablauf der halben Pulsdauer 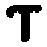 , wechselt der Strom I(t) im Schwingkreis seine Polarität und der Thyristor (T2) (Figur 7) wird durchgeschaltet und übernimmt die Stromleitung bis eine volle Sinusschwingung erreicht ist. Der Thyristor (T1) ist währenddessen gesperrt. Die hierdurch sich ergebende negative Halbwelle des sinusförmigen Stromverlaufs besitzt ebenfalls eine maximale Amplitude von circa 3000 A. Da nach einer Impulsdauer 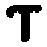 beide Thyristoren (T1, T2) gesperrt sind, wird eine weitere Schwingung unterbunden, so dass sich nur ein biphasiger Puls ergibt. Erst nach Erreichen einer Stimulationspulswiederholzeit **T**, die dem Kehrwert der Stimulationsfrequenz **f_{Stim}** entspricht, werden die Thyristoren (T1, T2) in der geschilderten Weise wieder durchgeschaltet und es ergibt sich der pulsförmige Stromverlauf in der Stimulationsspule aufs Neue, wie in Figur 8 ebenfalls gezeigt. Die dargestellten pulsförmigen Ströme sorgen dann in der Stimulationsspule (3) für einen entsprechenden Auf- und Abbau pulsförmiger magnetischer Wechselfelder innerhalb eines an der Bedienungskonsole (8) einstellbaren oder vom Computer (9) gesteuerten Behandlungszeitintervalls.

Wird der Thyristor T2 niemals durchgeschaltet, so ergibt sich ein monophasiger Strompuls, der nur aus der in Figur 8 dargestellten positiven Halbwelle des sinusförmigen Stromverlaufs I(t) besteht.

### Bezugszeichenliste

- 1: Ständer
- 2: Kunststoffgehäuse
- 3: Magnetfeldspule/Stimulationsspule
- 4: Podest
- 5: Fußhebel
- 6: Kamera
- 7: Einstellring
- 8: Bedienungskonsole
- 9: Laptop
- 10: Luftgebläse
- 11: Pulsgenerator
- 12: Kabelkanal
- 13: Strahler
- 14: Netzgerät
- 15: Verbindungkabel
- 16: Hohlrahmen
- 17: Halteelement
- 18: Verbindungsleitung
- 19: Verbindungsleitung
- 20: Luftführungskanal
- 21: Verschlusshülse
- 22: Lufteinlassöffnungen
- 23: Filtermatten
- 24: Windungen
- 25: Leitungskopplung
- 26: Endabschnitt
- 27: Außengewinde
- 28: Schlitz
- 29: Lasche
- 30: Endabschnitt
- 31: Schraubmuffe
- 32: Schutzdeckel
- 33: Nut
- 34: Luftauslassöffnung

Die vorliegende Erfindung nutzt den Effekt der Stimulation von Muskelkontraktionen durch berührungslose Induktion von elektrischen Feldern mittels pulsförmiger Magnetfelder im Gewebe. Pulsierende Magnetfelder sind dabei auch in der Lage durch Beeinflussung der elektrischen Ströme den Ionentransport anzuregen und den Stoffwechsel messbar zu erhöhen. Es kommt nachweislich zu einer Steigerung der Durchblutung und einer erhöhten Sauerstoffzufuhr. Diese Effekte werden derzeit unter dem Gesichtspunkt der Reaktivierung von Muskeln nach Krankheit oder Unfall medizinisch evaluiert und haben bereits zu entsprechenden Erfindungen geführt. So ist aus der DE 10 2007 044 445 A1 ein Trainingsgerät mit Magnetstimulation bekannt, bei dem in Verbindung mit Mitteln zur mechanischen Führung einer beabsichtigten Gelenkbewegung eines gelähmten Körperteils dieses zu Bewegungen angeregt wird. Aus der US 2005/0203332 A1 ist ein Gerät zur Behandlung von Osteoporose und anderen Muskel-Skelett-Erkrankungen bekannt, bei der der Patient auf einer Liege liegend am betreffenden Körperteil von einer zylindrischen, ein elektromagnetisches Feld erzeugenden Spule umgeben ist. Aus der US 6,213,933 B1 ist eine Vorrichtung und ein Verfahren zur Auflösung von Blutgerinseln in menschlichen Körperteilen entnehmbar, wobei der Patient auf einer länglichen Plattform liegt, über die eine quer verlaufende, längsbewegliche Halterung mit einer Wasser gekühlten Magnetfeldspule vom Butterfly Typ zur Stimulation angebracht ist. Zur Positionierung der Magnetfeldspule wird die Halterung einfach über das zu behandelnde Körperteil geschoben. Die Frequenz und Dauer der Stimulation wird mittels eines interaktiven Programms von einem PC gesteuert. Ein anderes, aus der US 200310158585 A1 bekanntes elektromagnetisches System nutzt zur Stimulation von Nerven, Muskeln und anderen Gewebeteilen des menschlichen Körpers ergonomische, der jeweiligen Körperteilkontur angepasste Stimulationsspulen in Form von flexiblen Flach- oder Zylinderspulen zur therapeutischen Behandlung.

Darüber hinausgehend - und bisher nicht genutzt -führt eine Muskelreizung mittels Magnetfeldstimulation zu einem Abbau von Fettgewebe in der Muskelumgebung, wie der Anmelder durch zahlreiche Versuche, insbesondere an adipösen und muskulösen Probanden, belegen konnte. Bei schlanken Probanden ergibt sich dabei ein Muskelaufbau ohne signifikante Gewichtsabnahme.

Zwar existieren bereits Vorrichtungen und Verfahren zur Behandlung von Fettleibigkeit bzw. Übergewicht mittels pulsierender Magnetfelder, diese benötigen aber neben der Feld erzeugenden Spule entweder noch einen mit der Körperoberfläche in Kontakt stehenden Permanentmagneten (DE 100 62 050 A1) oder wirken indirekt über ein die Schilddrüse aktivierendes Magnetfeld (DE 10 2009 043 728 A1) und müssen mittels eines Halsbandes ebenfalls am Körper angebracht werden.

Unter Ausnutzung der zuvor genannten Erfahrung und Erkenntnisse verbleibt somit als Aufgabe der Erfindung, eine Vorrichtung und ein Verfahren für repetitive Nervenstimulation zum Abbau von Fettgewebe mittels induktiver Magnetfelder zu schaffen, die eine leichte patientenbezogene Einstellbarkeit und Steuerung erlauben, um Fettgewebe in definierten Körperregionen, wie Bauch, Gesäß oder Oberschenkel, gezielt und ohne Körperkontakt zu reduzieren.

Diese Aufgabe wird durch die im Patentanspruch 1 beanspruchte Vorrichtung
gelöst. Vorteilhafte Ausgestaltungen der Erfindung sind Gegenstand von Unteransprüchen.

Vorteile der Erfindung bestehen insbesondere in der berührungslosen Induktion der Reizung, der damit verbundenen geringen Schmerzreizung, die bei alternativer elektrischer Reizung auftritt, der Großflächigkeit der Reizung und der auf den individuellen Körperbau des Patienten abstimmbaren Positionierung der reizerzeugenden Spule.

Wesentliche Bestandteile des Gerätes sind eine großflächige stromdurchflossen Magnetfeldspule, im Weiteren Stimulationsspule genannt, die auf einem Ständer befestigt ist. Für die Behandlung von Bauch, Gesäß und Oberschenkeln sind verschiedene Spulenformen vorhanden

Die Stimulationsspule erzeugt in ca. 5 cm vor der Spulenoberfläche Magnetfelder mit Spitzen in der magnetischen Flussdichte von 0.01 T bis 0,1 T. Das Magnetfeld ist dabei zeitlich veränderlich und besteht aus biphasigen oder monophasigen Pulsen mit einer Pulsdauer T von 100 µs bis 300 µs. Die Wiederholfrequenz der Pulse (Stimulationsfrequenz fₚ) beträgt 10 Hz bis 30 Hz. Mittels Induktion werden am Reizort im Gewebe maximale elektrische Feldstärken von 0.1 V/cm bis 1 V/cm erreicht. Das Magnetfeld der Spule ist betragsmäßig über das zu behandelnde Gewebevolumen näherungsweise örtlich konstant. Parameter einer typischen Spule sind in Tabelle 1 zusammengestellt.

**Tabelle 1: Übersicht zu den Parametern der Spule für den Bauchbereich**

| **Größe** | **Wert** |
|---|---|
| Länge des Leiters | 200 cm |
| Querschnittsfläche | 1 cm² |
| Abstand benachbarter Spulenleiter | 1 cm |
| Maximale Stromamplitude I | 1000 A |
| Max. magn. Flussdichte B, 5 cm vor der Spulenoberfläche | 0.01 T |
| Induzierte elektrische Feldstärke (maximal), E | 0.1 V cm⁻¹ |
| Kräfte zwischen benachbarten Spulenleitern | 0,2 Ncm⁻¹ |
| Induktivität der Spule, L | 15 10⁻⁶ H |
| Pulsfrequenz, f | 5 10³ Hz |
| Induktiver Widerstand Z_{L} bei f | 470 m Ohm |
| induktiver Spannungsabfall bei I | 470 V |
| Ohmscher Widerstand, R | 36 m Ohm |
| Stimulationsfrequenz, f_{Stim} | 30 Hz |
| Joulsche Verlustleistung, P | 120 W |

Die Ansteuerung der Stimulationsspule erfolgt über einen Pulsgenerator (Stimulator), der getrennt vom Ständer aufgestellt ist. Es kann ein derzeit als Prototyp in der medizinischen Forschung verwendeter Stimulator (IMETUM, Zentralinstitut für Medizintechnik, Technische Universität München, Abschlussbericht: "Funktionelle, periphere Magnetstimulation der Motorik in Patienten mit zentralen Paresen, insbesondere halbseitigen Lähmungen", 2011) mit einer Pulslänge von 160 µs (biphasig) eingesetzt werden. Die Kapazität eines Kondensators wird dabei an die Induktivität der Stimulationsspule angepasst, um die Resonanzfrequenz eines LC-Schwingkreises, bestehend aus der Stimulationsspule und dem Kondensator, auf die der Pulsdauer entsprechenden Frequenz abzustimmen. Die erforderlichen elektrischen Felder bedingen hohe Ströme durch die Spule im Bereich von 500 A bis 6000 A.

Die Großflächigkeit der Spule gestattet einen offenen und nicht vergossenen Aufbau. Dadurch kann die erhebliche anfallende Joulsche Wärme in der Spule mittels eines Luftgebläses abgeführt werden. Luftführung ist zur effektiven Kühlung vorgesehen. Mittels Filtermatten wird eine Kontamination und Staubverwirbelung vermieden.

Die Behandlungszeiten betragen 1 min bis 45 min. Die langen Zeiten können ohne Überhitzung der Spule aufgrund der effektiven Kühlung und der Spulenkonstruktion erreicht werden.

Die Spule selbst kann aufgrund ihrer Größe als selbsttragend aus massivem Metall (z.B. Kupfer oder Aluminium) gefertigt werden. Dadurch wird die Wärmeableitung aus dem Spulenleiter an seine Oberfläche unterstützt. Der Querschnitt des Leiters beträgt 1 cm² bis ca. 2 cm². Dieser im Vergleich zu dem von Spulen, die in der ortselektiven Magnetfeldstimulation (fokussierend) eingesetzt werden, große Querschnitt des Spulenleiters reduziert den ohmschen Widerstand, wodurch die Joulsche Wärme reduziert wird.

Alternativ kann die Spule zur Vermeidung von Skin- und Proximity-Effekten auch in konventioneller Technik aus Hochfrequenzdrähten erstellt werden. Eine Abschätzung der Skintiefe zeigt aber, dass dies bei einer Pulsdauer von ca. 200 µs nicht erforderlich ist.

Bei massiver Bauweise ist ggf. auch die Verwendung von Hohlleitern, die mittels einer Kühlflüssigkeit gekühlt werden denkbar.

Die Stimulationsspule ist in einem Gehäuse aus Kunststoff gekapselt. Das Gehäuse gewährleistet die Berührspannungssicherheit und gleichzeitig die Führung der Kühlluft.

Das Luftgebläse erzeugt einen Kühlluftstrom, der über im Kunststoffgehäuse der Stimulationsspule befindliche, mit Filter versehene Lufteintrittsöffnungen und Luftführungselemente an die Spule gelangt und über eine Luftführung aus Kunststoff, die um die elektrischen Verbindungsleitungen zur Spule angeordnet ist, den Luftaustrittsöffnungen des Kühlgebläses zugeführt wird. Das Luftgebläse kann dadurch rückseitig am Ständer und außerhalb des Magnetfeldes der Stimulationsspule angeordnet werden, wodurch eine Beeinträchtigung des elektrischen Gebläsemotors vermieden wird.

Aufgrund der hohen Ströme treten hohe mechanische Kräfte zwischen den Leiterabschnitten der Stimulationsspule und den Verbindungsleitungen auf. Diese werden durch abstandshaltende, keramische Halteelemente abgefangen.

Die elektrischen Verbindungsleitungen zur Stimulationsspule im Ständer sind massiv und ohne bewegliche Kabel ausgeführt. Dies erlaubt eine optimale Wärmeabgabe an die Luft, Abfangen der magnetischen Kräfte und eine hohe Betriebssicherheit.

Die Stimulationsspule ist auf einer Halterung montiert, die durch die massiven Stromzuführungen selbst gebildet wird. Diese positioniert die Spule in ausreichendem Abstand vom Ständer; die durch die Spule im Ständer induzierten, unerwünschten Wirbelströme werden so minimiert.

Eine Stimulationsspule kann nach Öffnung einer tordial und axial gelagerten Verschlusshülse mit Bajonettverschluss und Lösen zweier Schraubverbindungen für die Verbindungsleitungen leicht ausgewechselt werden. Dies ist relevant, da für unterschiedliche Körperregionen (Bauch, Oberschenkel, Gesäß) verschiedene, optimierte Spulenformen mit unterschiedlichen Gehäusen eingesetzt werden.

Die Positionierung der Stimulationsspule relativ zum Patienten kann mittels eines höhenvariablen Podestes variiert werden. Dadurch können die Verbindungsleitungen zur Stimulationsspule starr ausgeführt werden und Änderungen der Induktivität und Zuleitungswiderstände bei Veränderung der Positionierung vermieden werden.

Alternativ könnte eine Positionierung der Stimulationsspule am Ständer durch Klemmverschlüsse in vertikalen Schienen ausgeführt werden.

Die Stimulation der Muskelkontraktion kann mittels einer Rückkopplung optimiert werden. Dazu werden mittels einer im Ständer integrierten Kamera die durch eine Stimulationsspule ausgelösten Kontraktionen beobachtet und entsprechende Signale an einen Computer (z.B. Laptop) übertragen. Dieser steuert mittels eines entsprechenden Programms in Abhängigkeit der empfangenen Signale die am Ausgang des Pulsgenerators liegenden elektrischen Impulse bezüglich Pulsform, Pulsdauer, Pulsamplitude und Behandlungszeit.

Ein Ausführungsbeispiel der Erfindung, das weitere Vorteile und Besonderheiten erkennen lässt, wird in den Figuren 1 bis 8 dargestellt und im Folgenden näher beschrieben.

Es zeigen:
- Figur 1:: Gesamtansicht der Vorrichtung für repetitive Nervenstimulation zum Abbau von Fettgewebe mittels induktiver Magnetfelder
- Figur 2:: Detailansicht der Ständer/Stimulationsspulen-Verbindungsanordnung
(a) perspektivische Ansicht
(b) Schnittdarstellung
- Figur 3:: Leitungskopplung für Hin- und Rückleiter innerhalb der Ständer/Stimulationsspulen-Verbindungsanordnung
(a) Schnittdarstellung
(b) perspektivische Darstellung im gekoppelten Zustand
- Figur 4:: Stimulationsspule zur Bauchbehandlung
(a) perspektivische Gehäuseansicht von vorn (Behandlungsseite) mit geöffnetem Schutzdeckel
(b) perspektivische Gehäuseansicht von hinten (ständerseitig) mit sichtbaren Leitungskopplungen
- Figur 5:: Stimulationsspule zur Behandlung der Gesäßregion
(a) Gehäuseansicht von vorne (Behandlungsseite)
(b) Gehäuseansicht von der Seite
(c) Gehäuseansicht in Aufsicht
(d) Spulenverlauf im Inneren des Gehäuses vom Behandelnden aus gesehen
- Figur 6:: Stimulationsspule zur Oberschenkelbehandlung
(a) Gehäuseansicht von vorne (Behandlungsseite)
(b) Gehäuseansicht von der Seite
(c) Gehäuseansicht in Aufsicht
(d) Spulenveriauf im Inneren des Gehäuses vom Behandelnden aus gesehen
- Figur 7:: Prinzipschaltbild eines bipolaren Pulsgenerators
- Figur 8:: idealisierter Stromverlauf in einer Stimulationsspule bei bipolarer Ansteuerung durch den Pulsgenerator

Figur 1 zeigt in Gesamtansicht die beanspruchte Vorrichtung für repetitive Nervenstimulation zum Abbau von Fettgewebe mittels induktiver Magnetfelder mit einer an einem Ständer (1) auswechselbar befestigten, in einem Kunststoffgehäuse (2) aufgenommenen Stimulationsspule (3), einem höhenvariablen Podest (4) mit Fußpumpe (5) zur individuell auf den Körper eines Patienten einstellbaren Positionierung gegenüber der Stimulationsspule (3), einer im Ständer (1) integrierten schwenkbaren Kamera (6) mit Einstellring (7) zur Beobachtung ausgelöster Muskelkontraktionen und zur Rückkopplung an einen in einer Bedienwngskonsole (8) integrierten Laptop(9) mit Steuerungssoftware zur Computer-unterstützten Stimulationsoptimierung, einem Luftgebläse (10) zur Kühlung der Magnetfeldspule (3), einem Pulsgenerator (11) zur elektrischen Ansteuerung der Magnetfeldspule (3), einem abgeschirmten Kabelkanal (12) für die notwendigen elektrischen Verbindungsleitungen zwischen dem Ständer(1) und dem Pulsgenerator (11) bsw. der Sedienungskonsole (8) und einen Strahler (13) zur korrekten Ausleuchtung der von der Kamera (6) erfassten Körperregion. Es wird darauf hingewiesen, dass der Pulsgenerator (11) in vorteilhafter Weise direkt am Ständer (1) angeordnet werden kann, um Leitungsverluste zu minimieren. In diesen Fall entfällt mithin der dargestellte Kabelkanal (12). Ebenfalls dargestellt ist das zur Spannungsversorgung der Vorrichtung gehörende, aus Gründen der hohen Wärmeentwicklung separat gestaltete und aufgestellte Netzgerät (14) mit Ausgangsspannungen zwischen 500 und 1000 V und den zugehörigen Verbindungskabeln (15).

Der im oberen Bereich dem magnetischen Feldlinienverlauf annähernd angepasste Ständer (1) besteht im Wesentlichen aus einem aus Kunststoff gefertigten Hohlrahmen (16) mit im Inneren befindlichen keramischen Halteelementen (17) zur genauen Positionierung der beabstandeten Verbindungsleitungen (18, 19) mit einem Leitungsquerschnitt von 1,5 cm² (Fig. 2a, b). Diese Verbindungsleitungen (18, 19) übernehmen die Stromführungen zwischen Pulsgenerator (11) und Stimulationsspule (3). Sie können auch als Hohlleiter ausgebildet sein. Etwa in Höhe der Taille eines Patienten weist der Ständer (1) einen fest eingebauten, horizontal und quer durch den Ständer (1) verlaufenden rohrförmigen Luftführungskanal (20) mit den Verbindungsleitungen (18, 19) im Inneren auf. Die Verbindungleitungen (18, 19) sind vom Luftführungskanal (20) durch abgedichtete Bohrungen in den Ständer (1) geführt.

Figur 1 zeigt weiterhin die ebenfalls im Ständer (1) vorhandene Kamera (6), die mittels eines Einstellringes (7) so positioniert werden kann, dass die behandelte Körperregion zuverlässig erfasst wird. Zum Ausleuchten der von der Kamera (6) erfassten Körperregion dient ein Strahler (13) am oberen Ende des Ständers (1).

Das höhenvariable Podest (4) kann eine mechanische Hubvorrichtung aufweisen, die mittels Fußhebel (5) betrieben und arretiert wird. Denkbar ist eine vertikale Stütze. Auch können vertikal übereinander angeordnete Scheren mit einer horizontal dazwischenliegenden Gewindespindel zur Höhenveränderung geöffnet oder geschlossen werden, wobei die Gewindespindel von Hand oder von einem Elektromotor gedreht wird. Als weitere konstruktive Höhenverstellung des Podestes (4) kann ein hydraulischer Hebezylinder mit Fußpumpe gewählt werden.

Figur 2a zeigt die Ständer/Stimulationsspulen-Verbindungsanordnung im Detail mit dem Ständer (1), dem Luftführungskanal (20) und dem Luftgebläse (10) zur Kühlung der Verbindungsleitungen (18, 19) sowie der Stimulationsspule (3). Eine axial verschieb- und verdrehbar gelagerte Verschlusshülse (21) mit Bajonettverschluss deckt den Anfang des Luftführungskanals (20) und das Ende der Stimulationsspule (3) als Berührungsschutz der Leitungskopplungen ab.

Figur 2b zeigt in Schnittdarstellung die Ständer/Stimulationsspulen-Verbindungsanordnung. Der Luftführungskanal (20) im Hohlrahmen (16) des Ständers (1) weist an dem Patienten abgewandten Ende das Luftgebläse (10) mit Luftauslassöffnungen (34) auf, welches Luft über die Lufteinlassöffnungen (22) im Kunststoffgehäuse (2) der Stimulationsspule (3), Filtermatten (23), vorbei an nicht dargestellten Luftführungselementen, den Windungen (24) der Stimulationsspule (3) und den Verbindungsleitungen (18, 19) zur Kühlung absaugt. Auch ist die Leitungskopplung (25) für Hin- und Rückleiter der Verbindungsleitungen (18, 19) dargestellt.

Figur 3a zeigt die Leitungskopplung (25) für Hin- und Rückleiter im Schnitt. Jedes Ende eines Leiters in der Stimulationsspule (3) weist einen verdickten, zylindrischen Endabschnitt (26) mit Außengewinde (27) auf. In diesen Endabschnitt (26) ist ein horizontaler, trapezförmiger Schlitz (28) gefräst. In diesen greift eine trapezförmige Lasche (29) ein, welche von einem verdickten, zylinderförmigen Endabschnitt (30) einer der Verbindungsleitungen (18) oder (19) ausgeht. Eine Schraubmuffe (31) mit Innengewinde, welche jeweils einen der verdickten Endabschnitte (30) der Verbindungsleitungen (18) oder (19) umgreift, ist auf das Außengewinde (27) jeweils eines Endabschnittes (26) aufgeschraubt und verbindet die Verbindungsleitungen (18, 19) mit der Stimulationsspule (3). Durch diese zwei steckbaren und fest verschraubbaren Leitungskopplungen wird die Stimulationsspule (3) von den Verbindungsleitungen (18, 19) getragen. Diese spezielle Schraubverbindung hat gleichzeitig die Funktion des elektrischen Leitens. Da die konischen Flanken des trapezförmigen Schlitzes (28) an den Flanken der trapezförmigen Lasche (29) großflächig anliegen, ergibt sich zudem ein geringer Übergangswiderstand.

Figur 3b zeigt die für Hin- und Rückleiter verwendete Leitungskopplung im verschraubten Zustand in perspektivischer Darstellung.

Figur 4a zeigt beispielsweise die Stimulationsspule (3) zur Bauchbehandlung in perspektivischer Darstellung von vorn (Behandlungsseite). Zu erkennen ist ein abgenommener aufklemmbarer Schutzdeckel (32) und ein annähernd trompetenförmiges Kunststoffgehäuse (2). An der Innenseite dieses trompetenförmigen Kunststoffgehäuses (2) sind mehrere keramische Halteelemente (17) zur Aufnahme der Windungen (24) der Stimulationsspule (3) vorhanden. Die Enden der Windungen (24) der Stimulationsspule (3) sind parallel geführt zu den verdickten Endabschnitten (26). Die Windungen (24) für die Bauchbehandlung selbst verlaufen annähernd oval und decken einen Bereich von ca. 20 cm x 30 cm ab. Auch sind die Lufteinlassöffnungen (22) erkennbar.

Figur 4b zeigt die Stimulationsspule (3) zur Bauchbehandlung in perspektivischer Darstellung von hinten (ständerseitig). Deutlich erkennbar sind die verdickten Endabschnitte (26) mit den Außengewinden (27), den jeweiligen trapezförmigen Schlitzen (28), keramische Halteelemente (17) und eine Nut (33) im Kunststoffgehäuse (2) der Stimulationsspule (3) zum Führen der axialen und verdrehenden Bewegung der Verschlusshülse (21).

Figuren 5a, 5b, 5c, 5d zeigen verschiedene Darstellungen einer Stimulationsspule (3) zur Behandlung der Gesäßregion; (a) die Gehäuseansicht von vorn, (b) die Gehäuseansicht von der Seite, (c) die Gehäuseansicht in Aufsicht und (d) den Spulenverlauf im inneren des Gehäuses vom Behandelten aus gesehen. Diese Stimulationsspule (3) hat den gleichen konstruktiven inneren Aufbau wie die Stimulationsspule (3) zur Bauchbehandlung und unterscheidet sich im Wesentlichen nur durch die äußere Form des Gehäuses. Die Behandlungsseite des Gehäuses hat eine abgerundete quadratisch Form von ca. 15 cm x 15 cm. Der Schutzdeckel (32) ist leicht konkav gewölbt. Die Simulatiohsspule (3) selbst besitzt ebenfalls quadratisch verlaufende, angepasste Windungen (24). Die Rückseite des Kunststoffgehäuses (2) ist schwanenhalsförmig geformt, damit die Stimulationsspule (3) ein Stück unterhalb der Taille des Patienten wirkt.

Figuren 6a, 6b, 6c, 6d zeigen verschiedene Darstellungen einer Stimulationsspule (3) zur Oberschenkelbehandlung; (a) die Gehäuseansicht von vorn, (b) die Gehäuseansicht von der Seite, (c) die Gehäuseansicht in Aufsicht und (d) Spulenverlauf im Inneren des Gehäuses vom Behandelten aus gesehen. Grundsätzlich ist diese Stimulationsspule (3) aufgebaut wie die für die Gesäßregion mit dem Unterschied, dass die Behandlungsseite eine abgerundete rechteckige Form von ca 10 cm x 20 cm abdeckt, wobei der Schutzdeckel eine in vertikaler Richtung verlaufende, leicht konkave Wölbung aufweist.

Figur 7 zeigt beispielhaft ein Prinzipschaltbild eines verwendeten herkömmlichen Pulsgenerators (11), der erst in Verbindung mit der Stimulationsspule (L) (siehe Leitungskopplungen LK) eine eigentliche Stimulationspulserzeugungseinheit ergibt. Vom separaten Netzteil (14) kommende Spannungen zwischen 500 V und 1000 V werden in Stufen von 50 V mittels elektronischer Schalter (hier nicht dargestellt), die entweder per Hand von der Bedienungskonsole (8) aus oder vom Computer (9) gesteuert werden, selektiert und an den am Eingang des Pulsgenerators (11) liegenden Ladekondensator (C1) zur Speicherung angelegt. Die gewählte Aufladspannung dieses Ladekondensators (C1) bestimmt im Wesentlichen die Stimulationsleistung und damit die Amplitudenhöhe des zu erzeugenden Stimulationspulses. Über die Thyristorschaltungen A und B wird der eigentliche Energiespeicher, der Pulskondensator (C2), auf- bzw. nachgeladen. Der Pulskondensator (C2) bildet mit der Stimulationsspule (L) im Prinzip einen Schwingkreis, der noch durch die ohmschen Widerstände der Verbindungsleitungen und -kopplungen, hier als ohmscher Widerstand (R) pauschal eingezeichnet, gedämpft wird. Repetitive Entladungen des Pulskondensators (C2) und damit die Stimulationsfrequenz **f_{Stim}** werden mittels zweier antiparallel geschalteter, starkstromtauglicher Thyristoren (T1, T2) gesteuert, die den Pulskondensator (C2) mit der Stimulationsspule (L) verbinden. Aufgrund der antiparallelen Schaltung der Thyristoren (T1, T2) können sinusförmige biphasige Spannungs- und Strompulse generiert werden (Figur 8 und zugehörige Beschreibung). Deren Impulsdauer 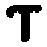 ist jeweils durch das Hinzu- oder Abschalten von zum Pulskondensator (C2) parallel geschalteten Kondensatoren (hier nicht dargestellt) einstellbar, da sich hierdurch die im Schwingkreis befindliche Gesamtkapazität und mithin die Schwingkreisfrequenz f_{P} (f_{P} = 1/(2π √LC) ändern lässt. Sowohl die Thyristoransteuerung zur Auswahl der Stimulationsfrequenz f_{Stim} als auch die Anzahl parallel geschalteter Kondensatoren zur Bestimmung der Pulsdauer 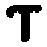 sind an der Bedienungskonsole (11) per Hand voreinstellbar oder werden vom Computer (9) kontrolliert.

In Figur 8 ist beispielhaft ein idealisierter Stromverlauf in einer Stimulationsspule (3) bei biphasiger Ansteuerung durch den Pulsgenerator (11) dargestellt. Hierbei wurde eine Stimulationsspuleninduktivität von 15×10⁻⁶ H, eine Aufspannung U_{C2} von 1000 V und ein biphasiger Stimulationspuls mit einer Pulsdauer T von 300 µs angenommen. Nach dem Durchschalten des Thyristors (T1) (Figur 7) ergibt sich eine positive Halbwelle eines sinusförmigen Stromverlaufs mit einer maximalen Amplitude von circa 3000 A. Zum Zeitpunkt 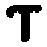 /2, also nach Ablauf der halben Pulsdauer 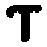 , wechselt der Strom I(t) im Schwingkreis seine Polarität und der Thyristor (T2) (Figur 7) wird durchgeschaltet und übernimmt die Stromleitung bis eine volle Sinusschwingung erreicht ist. Der Thyristor (T1) ist währenddessen gesperrt. Die hierdurch sich ergebende negative Halbwelle des sinusförmigen Stromverfaufs besitzt ebenfalls eine maximale Amplitude von circa 3000 A. Nach einer Impulsdauer 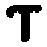 sind beide Thyristoren (T1, T2) gesperrt. Dadurch wird eine weitere Schwingung unterbunden. Folglich ergibt sich nur ein biphasiger Puls. Erst nach Erreichen einer Stimulationspulswiederholzeit T. die dem Kehrwert der Stimulationsfrequenz **f_{Stim}** entspricht, werden die Thyristoren (T1. T2) in der geschilderten Weise wieder durchgeschaltet und es ergibt sich der pulsförmige Stromverlauf in der Stimulationsspule aufs Neue, wie in Figur 8 ebenfalls gezeigt. Die dargestellten pulsförmigen Ströme sorgen dann in der Stimulationsspule (3) für einen entsprechenden Auf- und Abbau pulsförmiger magnetischer Wechselfelder innerhalb eines an der Bedienungskonsole (8) einstellbaren oder vom Computer (9) gesteuerten Behandlungszeitintervalls.

Wird der Thyristor T2 niemals durchgeschaltet, so ergibt sich ein monophasiger Strompuls, der nur aus der in Figur 8 dargestellten positiven Halbwelle des sinusförmigen Stromverlaufs I(t) besteht.

### Bezugszeichenliste

- 1: Ständer
- 2: Kunststoffgehäuse
- 3: Magnetfeldspule/Stirmulationsspule
- 4: Podest
- 5: Fußhebel
- 6: Kamera
- 7: Einstellring
- 8: Bedienungskonsole
- 9: Laptop
- 10: Luftgebläse
- 11: Pulsgenerator
- 12: Kabelkanal
- 13: Strahler
- 14: Netzgerät
- 15: Verbindungkabel
- 16: Hohlrahmen
- 17: Halteelement
- 18: Verbindungsleitung
- 19: Verbindungsleitung
- 20: Luftführungskanal
- 21: Verschlusshülse
- 22: Lufteinlassöffnungen
- 23: Filtermatten
- 24: Windungen
- 25: Leitungskopplung
- 26: Endabschnitt
- 27: Außengewinde
- 28: Schlitz
- 29: Lasche
- 30: Endabschnitt
- 31: Schraubmuffe
- 32: Schutzdeckel
- 33: Nut
- 34: Luftauslassöffnung

## Patentansprüche

1. Vorrichtung für repetitive Nervenstimulation zum Abbau von Fettgewebe mittels induktiver Magnetfelder mit einer an einem Ständer (1) auswechselbar befestigten, in einem Kunststoffgehäuse (2) aufgenommenen Stimulationsspule (3), einem höhenvariablen Podest (4) zur individuell auf den Körper eines Patienten einstellbaren Positionierung gegenüber der Stimulationsspule (3), einem Luftgebläse (10) zur Kühlung der Stimulationsspule (3), einem Pulsgenerator (11) zur elektrischen Ansteuerung der Stimulationsspule (3) sowie ein separates Netzgerät (14) zur Generierung der Veraorgungsapannungen.

2. Vorrichtung nach Patentanspruch 1, **gekennzeichnet durch** eine im Ständer (1) integrierte schwenkbare Kamera (6) zur Beobachtung ausgelöster Muskelkontraktionen und zur Rückkopplung an einen in einer Bedienungskonsole (8) integrierten Laptop (9) mit Steuerungssoftware zur Computer-uriterstützten Stimulationsoptimierung

3. Vorrichtung nach Patentanspruch 2, **dadurch gekennzeichnet, dass** die im Ständer (1) integrierte Kamera (6) mittels eines Einstellringes (7) von Hand schwenkbar ist.

4. Vorrichtung nach einem der vorangehenden Patentansprüche, **gekennzeichnet durch** einen Satz von verschiedenen Stimulstionsspulen (3), die auf unterschiedliche Körperbereiche, wie Bauch. Gesäß oder Oberschenkel in Form und Größe angepasst sind.

5. Vorrichtung nach einem der vorhergehenden Patentansprüche, **dadurch gekennzeichnet, dass** der Leiter der Stimulationsspule (3) und im Ständer (1) befindliche, zugehörige Verbindungsleitungen (18, 19) mit dem Pulsgenerator (11) aus einem massiven Metall, belspielsweise Kupfer oder Aluminium, mit einem Leiterquerachnitt von 1 cm² bis 2 cm² bestehen und eine tragende Funktion für die Stimulationsspule (3) in der Vorrichtung übernehmen.

6. Vorrichtung nach Patentanspruch 5, **dadurch gekennzeichnet, dass** Leitungskopplungen (25) zwischen einer der Verbindungsleitungen (18, 19) und einem Endabschnitt (26) eines Stimulationsspulenleiters aus einer im Querschnitt trapezförmig ausgestalteten Steckverbindung (28, 29) und einer Schraubmuffe (31) bestehen.

7. Vorrichtung nach Patentanspruch 5, **dadurch gekennzeichnet, dass** keramische Halteelemente (17) als Abstandshalter sowohl zwischen den Windungen der Stimulationsspule (3) als auch zwischen den Verbindungsleitungen (18, 19) und dem jeweils sie umgebenden Gehäuse (2) oder Ständer (1) dienen.

8. Vorrichtung nach einem der vorhergehenden Patentansprüche, **dadurch gekennzeichnet, dass** das Kunststoffgehäuse (2) der Stimulationsspule (3) Lufteinlassöffnungen (22) und Luftführungselemente im Inneren aufweist, damit geführte Luft zur Kühlung der Stimulationsspule (3) durch einen die Verbindungsleitungen (18, 19) umgebenden Luftführungskanal (20) des Ständers (1) vom Luftgebläse (10) abgesaugt werden kann.

9. Vorrichtung nach Patentanspruch 8, **dadurch gekennzeichnet, dass** an der Innenseite des Kunststoffgehäuses (2) im Bereich der Lufteinlassöffnungen (22) Filtermatten (23) vorhanden sind:

10. Vorrichtung nach einem der vorhergehenden Patentansprüche, **dadurch gekennzeichnet, dass** das höhenvariable Podest (4) eine mechanische Hubvorrichtung aufweist, die eine vertikalbewegliche Stütze mit einrastbaren Arretierungen oder vertikal übereinander angeordneten Scheren mit einer horizontal dazwischen ilagenden, motorisch oder per Handkurbel angetriebenen Gewindespindel oder einen Fußpumpen betätigten hydraulischen Hebezylinder beinhaltet

11. Vorrichtung nach einem der vorhergehenden Patentansprüche 2 bis 10, **dadurch gekennzeichnet, dass** im Ständer (1) ein Strahler (13) zur korrekten Ausleuchtung der von der Kamera (6) erfassten Körperregion zur Aufnahme von Muskelkontraktionen integriert ist.

12. Vorrichtung nach einem der vorhergehenden Patentansprüche, **dadurch gekennzeichnet, dass** die Form des Ständers (1) teilweise dem magnetischen Feldlinierivertauf angepasst ist.

13. Vorrichtung nach einem der vorhergehenden Patentansprüche, **dadurch gekennzeichnet, dass** der Pulsgenerator (11) direkt am Ständer (1) angeordnet ist.

## Claims

1. Device for repetitive nerve stimulation for breaking down fatty tissue by means of inductive magnetic fields, having a stimulation coil (3) replaceably attached to a stand (1) and accommodated in a plastic housing (2), a variable-height pedestal (4) for adjustable positioning relative to the stimulation coil (3) to tailor it individually to a patient's body, a fan (10) for cooling the stimulation coil (3), a pulse generator (11) for electrically actuating the stimulation coil (3) and a separate power unit (14) for generating the supply voltages.

2. Device according to claim 1, **characterised by** a pivotable camera (6) integrated in the stand (1) for observing muscle contractions produced and for feeding back to a laptop (9) integrated in an operating console (8), with control software for computer-aided optimisation of stimulation.

3. Device according to claim 2, **characterised in that** the camera (6) integrated in the stand (1) is manually pivotable by means of an adjustment ring (7).

4. Device according to one of the preceding claims, **characterised by** a set of different stimulation coils (3) which are adapted in shape and size to different areas of the body such as the abdomen, buttocks or thighs.

5. Device according to one of the preceding claims, **characterised in that** the conductor of the stimulation coil (3) and associated connecting leads (18, 19) to the pulse generator (11) which are located in the stand (1) consist of a solid metal, for example copper or aluminium, with a conductor cross-section of 1 cm² to 2 cm² and assume a supporting function for the stimulation coil (3) in the device.

6. Device according to claim 5, **characterised in that** lead couplings (25) between one of the connecting leads (18, 19) and an end portion (26) of a stimulation coil conductor consist of a plug-in connection (28, 29) of trapezoidal cross-section and a screw bushing (31).

7. Device according to claim 5, **characterised in that** ceramic retaining elements (17) serve as spacers both between the windings of the stimulation coil (3) and also between the connecting leads (18, 19) and the housing (2) or stand (1) surrounding them.

8. Device according to one of the preceding claims, **characterised in that** the plastic housing of the stimulation coil (3) comprises air inlet openings (22) and air guiding elements inside it so that the air it carries for cooling the stimulation coil can be aspirated by the fan through an air conveying channel (20) of the stand (1) surrounding the connecting leads (18, 19).

9. Device according to claim 8, **characterised in that** filter mats (23) are provided on the inside of the plastic housing (2) in the region of the air inlet openings (22).

10. Device according to one of the preceding claims, **characterised in that** the variable-height pedestal (4) comprises a mechanical lifting device which contains a vertically movable upright with engageable locking means, or scissor-type supports arranged vertically above one another with a threaded spindle located horizontally between them, motor-driven or hand-cranked, or a hydraulic lifting cylinder operated by a foot pump.

11. Device according to one of the preceding claims 2 to 10, **characterised in that** integrated in the stand (1) is a spotlight (13) for the correct illumination of the area of the body captured by the camera (6) in order to record muscle contractions.

12. Device according to one of the preceding claims, **characterised in that** the shape of the stand (1) is partially adapted to the magnetic field line pattern.

13. Device according to one of the preceding claims, **characterised in that** the pulse generator (11) is arranged directly on the stand (1).

## Revendications

1. Installation de stimulation nerveuse répétitive pour réduire des tissus adipeux au moyen de champs magnétiques inductifs avec une bobine de stimulation (3) fixée de manière interchangeable à un pilier (1) reçue dans un boîtier en matière plastique (2), une estrade (4) variable en hauteur pour le positionnement réglable individuellement en fonction du corps d'un patient par rapport à la bobine de stimulation (3), un ventilateur (10) pour le refroidissement de la bobine de stimulation (3), un générateur d'impulsion (11) pour la commande électrique de la bobine de stimulation (3) ainsi qu'un bloc d'alimentation (14) séparé pour la génération des tensions d'alimentation.

2. Installation selon la revendication 1, **caractérisée par** une caméra (6) pouvant pivoter intégrée dans le pilier (1) pour l'observation de contractions musculaires déclenchées et pour la rétroaction sur un ordinateur portable (9) intégré dans une console d'opération (8) avec un logiciel de commande pour l'optimisation de la stimulation assistée par ordinateur.

3. Installation selon la revendication 2, **caractérisée en ce que** la caméra (6) intégrée dans le pilier (1) peut être pivotée manuellement au moyen d'un anneau de réglage (7).

4. Installation selon l'une quelconque des revendications précédentes, **caractérisée par** un ensemble de bobines de stimulation (3) diverses qui sont adaptées à différentes zones corporelles, telles que le ventre, le fessier ou les cuisses en termes de forme et de taille.

5. Installation selon l'une quelconque des revendications précédentes, **caractérisée en ce que** le conducteur des impulsions de stimulation (3) et des câbles de raccordement (18, 19) correspondants se trouvant dans le pilier (1) avec le générateur d'impulsions (11) sont constitués d'un métal massif, par exemple du cuivre ou de l'aluminium, avec une section de conducteur de 1 cm² à 2 cm² et assurent une fonction porteuse pour la bobine de stimulation (3) dans l'installation.

6. Installation selon la revendication 5, **caractérisée en ce que** des couplages de conduction (25) entre l'un des câbles de raccordement (18, 19) et une partie d'extrémité (26) d'un conducteur de bobine de stimulation sont constitués d'un raccord enfichable (28, 29) conçu avec une section trapézoïdale et d'un manchon à visser (31).

7. Installation selon la revendication 5, **caractérisée en ce que** des éléments de retenue (17) en céramique servent d'espaceurs à la fois entre les enroulements de la bobine de stimulation (3) et également entre les câbles de raccordement (18, 19) et le boîtier (2) les entourant respectivement ou le pilier (1).

8. Installation selon l'une quelconque des revendications précédentes, **caractérisée en ce que** le boîtier en matière plastique (2) de la bobine de stimulation (3) présente des entrées d'admission d'air (22) et des éléments de conduction d'air à l'intérieur, afin que de l'air acheminé pour le refroidissement de la bobine de stimulation (3) puisse être aspiré au travers d'un canal d'acheminement d'air (20) du pilier (1) entourant les câbles de raccordement (18, 19) par le ventilateur (10).

9. Installation selon la revendication 8, **caractérisée en ce que** sur le côté interne du boîtier en matière plastique (2) dans la zone des entrées d'admission d'air (22), des nattes de filtrage (23) sont présentes.

10. Installation selon l'une quelconque des revendications précédentes, **caractérisée en ce que** l'estrade (4) variable en hauteur présente une installation de levage mécanique qui comporte un support mobile verticalement avec des arrêts pouvant être encliquetés ou des ciseaux disposés verticalement les uns au-dessus des autres avec une broche filetée se trouvant horizontalement entre elles, entraînée par un moteur ou une manivelle ou un cylindre de levage hydraulique actionné par une pompe à pied.

11. Installation selon l'une quelconque des revendications 2 à 10, **caractérisée en ce que** dans le pilier (1) est intégré un projecteur (13) pour l'éclairage correct de la région corporelle saisie par la caméra (6) pour l'enregistrement de contractions musculaires.

12. Installation selon l'une quelconque des revendications précédentes, **caractérisée en ce que** la forme du pilier (1) est partiellement adaptée au parcours de ligne de champ magnétique.

13. Installation selon l'une quelconque des revendications précédentes, **caractérisée en ce que** le générateur d'impulsions (11) est disposé directement au niveau du pilier (1).
